# EUROPEAN PATENT SPECIFICATION

(11) **EP 3 989 823 B1**
(45) Date of publication and mention of the grant of the patent: **02.08.2023**
(21) Application number: 20734748.5
(22) Date of filing: 29.06.2020
(51) Int. Cl.: A61B 5/1455, G01N 21/84, G01N 21/78

(54) **DETERMINATION OF A CONCENTRATION OF AN ANALYTE IN A BODILY FLUID BY USING A MOBILE DEVICE WITH A CAMERA**
BESTIMMUNG DER KONZENTRATION EINES ANALYTEN IN EINER KÖRPERFLÜSSIGKEIT UNTER VERWENDUNG EINES MOBILGERÄTS MIT EINER KAMERA
DÉTERMINATION DE LA CONCENTRATION D'UN ANALYTE DANS UN FLUIDE CORPOREL EN UTILISANT UN APPAREIL MOBILE MUNI D'UNE CAMERA

(30) Priority: 01.07.2019 EP 19183592
(43) Date of publication of application: 04.05.2022
(73) Proprietor: F. Hoffmann-La Roche AG, 4070 Basel (CH); Roche Diabetes Care GmbH, 68305 Mannheim (DE)
(72) Inventor: LIMBURG, Bernd, 68305 Mannheim (DE); BERG, Max, 68305 Mannheim (DE); HAILER, Fredrik, 68305 Mannheim (DE)
(74) Representative: Riwotzki, Karsten
(86) International application number: PCT/EP2020/068179
(87) International publication number: WO 2021/001285

(56) References cited:
- EP-A1- 1 801 568
- EP-A1- 3 477 270
- WO-A1-2012/131386
- WO-A1-2018/166533

## Description

### Technical Field

The present invention refers to a method of determining a concentration of an analyte in a bodily fluid by using a mobile device having a camera. The invention further relates to a mobile device having a camera, to a kit comprising the mobile device and at least one test element, and a computer-readable storage medium. The invention specifically may be used in medical diagnostics, in order to for example qualitatively or quantitatively detect one or more analytes in one or more bodily fluids, such as for blood glucose measurements. Other fields of application of the present invention, however, are possible.

### Background art

In the field of medical diagnostics, in many cases, one or more analytes have to be detected in samples of a body fluid, such as blood, interstitial fluid, urine, saliva or other types of body fluids. Examples of analytes to be detected are glucose, triglycerides, lactate, cholesterol or other types of analytes typically present in these body fluids. According to the concentration and/or the presence of the analyte, an appropriate treatment may be chosen, if necessary. Without narrowing the scope, the invention specifically will be described with respect to blood glucose measurements. It shall be noted, however, that the present invention may also be used for other types of analytical measurements using test elements such as test strips.

Generally, devices and methods known to the skilled person make use of test strips comprising one or more test chemistries, which, in the presence of the analyte to be detected, are capable of performing one or more detectable detection reactions, such as optically detectable detection reactions. With regard to these test chemistries, reference may be made e.g. to J. Hoenes et al.: The Technology Behind Glucose Meters: Test Strips, Diabetes Technology & Therapeutics, Volume 10, Supplement 1, 2008, S-10 to S-26. Other types of test chemistries are possible and may be used for performing the present invention.

Typically, one or more optically detectable changes in the test chemistry are monitored, in order to derive the concentration of the at least one analyte to be detected from these changes. For detecting the at least one change of optical properties of the test field, various types of detectors, specifically customized detectors, are known in the art. Thus, various types of light sources for illuminating the test fields as well as various types of detectors are known.

Further, besides using customized detectors which are specifically developed for the purpose of optically detecting changes in the test chemistry comprised by corresponding test elements, recent developments aim at using widely available devices such as smartphones. However, when consumer-electronics devices having a camera, such as smartphones, are employed in order to determine analyte concentrations new challenges, in particular concerning the accuracy, arise.

US 9892505 B2 discloses a method for determining vital parameters of a human body via a device, in particular a smart device, said device comprising an optical recording unit and a computing unit. The method comprises recording a sequence of individual image data of limited area of the skin of the human body via the optical recording unit.

EP 2646809 B1 discloses a testing apparatus for performing an assay. The testing apparatus comprises a receptacle containing a reagent, the reagent being reactive to an applied test sample by developing a colour or pattern variation. The testing apparatus further comprises a portable device comprising a processor and an image capture device. The processor is adapted to correct the image for any rotational misalignment or skew. The processor further is adapted to determine a degree of error associated with any rotational misalignment or skew for correcting the image. The processor is configured to process data captured by the image capture device and output a test result for the applied test sample. The testing apparatus is configured to reject an image when a degree of error associated with any rotational misalignment or skew is greater than a predetermined value.

US 10267743 B2 discloses a method for quantifying color change of at least one test medium on a diagnostic instrument. The method comprises capturing, with a digital camera, capturing a digital image of at least a portion of the diagnostic instrument that has been exposed to a biological sample, the diagnostic instrument comprising at least one color reference including a plurality of reference samples of different colors and at least one test medium containing a reagent that changes color in response to concentration of a particular analyte in the biological sample. The method further comprises identifying at least one reference sample of the plurality of reference samples for the at least one test medium in the diagnostic instrument and determining a dominant camera-captured color of the at least one reference sample and a dominant camera-captured color of the at least one test medium. The method further comprises estimating lighting conditions under which the digital image is captured. The method further comprises correcting the dominant camera-captured color of the at least one test medium, in response to a color correction factor derived at least in part from the dominant camera-captured color of the at least one reference sample, to determine a corrected test medium color. The method finally comprises determining a test result including an analyte concentration of the biological sample by comparing the corrected test medium color to a set of possible test medium colors corresponding to predetermined analyte concentrations, wherein the set of possible test medium colors is responsive to the estimated lighting conditions under which the digital image is captured.

WO 2012/131386 A1 discloses a testing apparatus for performing an assay, the testing apparatus comprising: a receptacle containing a reagent, the reagent being reactive to an applied test sample by developing a color or pattern variation; a portable device, e.g. a mobile phone or a laptop, comprising a processor and an image capture device, wherein the processor is configured to process data captured by the image capture device and output a test result for the applied test sample.

WO 2018/166533 A1 describes example methods to improve placement of an adaptor to a mobile computing device to measure a test strip coupled to the adaptor with a camera and a screen on a face of the mobile computing device. The method may include displaying a light area on a first portion of the screen. The first portion may be adjacent to the camera. The light area and the camera may be aligned with a key area of the test strip so that the camera is configured to capture an image of the key area. The method may further include providing first guiding information for a user to place the adaptor to the mobile computing device according to a position of the light area on the screen.

EP 1 801 568 A1 discloses a method for positioning a camera at a test strip for pictorially detecting a color indicator and a reference color area. A measured value is determined for the relative position between the camera and the strip and compared with a desired value area. The camera is moved to reduce deflection relative to the strip during the deflection between the measured value and the desired value. An image area assigned to the indicator is localized in a colored image that is detected by the camera. An analyte concentration is determined in a sample by a comparison value. Independent claims are also included for the following: a computer program that is executed on a camera that is programmed by a microprocessor a server with a microcomputer that includes a memory a test strip for determining an analyte concentration in a sample of a biological fluid.

EP 3 477 270 A1 describes a method for evaluating the suitability of a mobile device having at least one camera for the purpose of performing an analytical measurement based on a color formation reaction. The method comprises: providing the at least one mobile device having the at least one camera; providing at least one object having at least one reference color field; taking at least one image of at least part of the reference color field by using the camera; and deriving at least one item of color resolution information by using the image.

US 9,311,520 B2 discloses methods and electronic devices for performing color-based reaction testing of biological materials. The method includes capturing and interpreting digital images of an unexposed and later exposed paddle at various delay times within an automatically calibrated environment. The test paddle includes a unique identification mechanism (UID), a Reference Color Bar (RCB) providing samples of standardized colors for image color calibration, compensation and corrections, and several test-specific sequences of Chemical Test Pads (CTP). The method further includes locating the paddle in the image, extracting the UID and validating the paddle, extracting the RCB and locating the plurality of CTP in each image. The method further reduces image noise in the CTP and calibrates the image automatically according to lighting measurements performed on the RCB. To determine test results, the method further determines several distances between the CTP and its possible trajectory in the color space described by the Manufacturer Interpretation Color Chart.

US 9,285,323 B2 describes a method for color quantification of chemical test pads and titration of analytes which can be performed under different lighting conditions. In one embodiment, the lighting condition is estimated under which a digital image is captured and utilized to select a set of reference colors from which the quantified color is compared to determine the titration. In another embodiment, a plurality of comparisons are made with different lighting conditions with the result having the highest confidence level being selected to determine the titration.

Despite the advantages involved in using consumer electronics having a camera for the purpose of detecting an analyte in a sample or evaluating analytical measurements, several technical challenges remain, specifically in view of measurement accuracy. These challenges mainly due to the fact that the measurements, when using consumer-electronics, typically take place under varying environmental and geometrical conditions, as opposed to measurements performed under laboratory conditions or measurements performed by using customized analytical measurement devices. Specifically, the varying conditions of illumination and reflection of light remain to be an issue and remain a major factor to be considered for increasing measurement accuracy. Still, when using custom electronics, fast and simple algorithms and procedures are required, in order to take account of the limited resources of consumer electronics devices and in order to avoid complicated and inconvenient measurement steps. Thus, elaborated correction algorithms typically are to be avoided in measurements taking place in the field by using consumer electronics devices.

### Problem to be solved

It is therefore desirable to provide methods and devices which address the above-mentioned shortcomings of known devices and methods. Specifically, a method of determining a concentration of at least one analyte in a body fluid shall be proposed, as well as corresponding devices, which provide for fast and efficient and still accurate measurements even under changing measurement conditions.

### Summary

This problem is addressed by the method of determining a concentration of an analyte in a bodily fluid by using a mobile device having a camera, by the mobile device having a camera, and by the kit comprising the mobile device and at least one test element, with the features of the independent claims. Advantageous embodiments which might be realized in an isolated fashion or in any arbitrary combinations are listed in the dependent claims.

As used in the following, the terms "have", "comprise" or "include" or any arbitrary grammatical variations thereof are used in a non-exclusive way. Thus, these terms may both refer to a situation in which, besides the feature introduced by these terms, no further features are present in the entity described in this context and to a situation in which one or more further features are present. As an example, the expressions "A has B", "A comprises B" and "A includes B" may both refer to a situation in which, besides B, no other element is present in A (i.e. a situation in which A solely and exclusively consists of B) and to a situation in which, besides B, one or more further elements are present in entity A, such as element C, elements C and D or even further elements.

Further, it shall be noted that the terms "at least one", "one or more" or similar expressions indicating that a feature or element may be present once or more than once typically will be used only once when introducing the respective feature or element. In the following, in most cases, when referring to the respective feature or element, the expressions "at least one" or "one or more" will not be repeated, non-withstanding the fact that the respective feature or element may be present once or more than once.

Further, as used in the following, the terms "preferably", "more preferably", "particularly", "more particularly", "specifically", "more specifically" or similar terms are used in conjunction with optional features, without restricting alternative possibilities. Thus, features introduced by these terms are optional features and are not intended to restrict the scope of the claims in any way. The invention may, as the skilled person will recognize, be performed by using alternative features. Similarly, features introduced by "in an embodiment of the invention" or similar expressions are intended to be optional features, without any restriction regarding alternative embodiments of the invention, without any restrictions regarding the scope of the invention and without any restriction regarding the possibility of combining the features introduced in such way with other optional or non-optional features of the invention.

In a first aspect of the present invention a method of determining a concentration of at least one analyte in a bodily fluid by using a mobile device and a test element, the mobile device having a camera, and the test element having at least one test field is proposed. The method comprises the following steps, which may specifically be performed in the given order. Still, a different order may also be possible. It may further be possible to perform two or more of the method steps fully or partially simultaneously. It may further be possible to perform one or more method steps or even all of the method steps once or repeatedly. The method may comprise additional method steps which are not listed herein. Generally, the method of determining a concentration of an analyte in a bodily fluid by using a mobile device having a camera comprises the following steps carried out by the mobile device:
a) determining an angular orientation of the mobile device relative to the test element by using sensor data of at least one sensor device integrated into the mobile device;
b) subjecting the angular orientation of the mobile device relative to the test element to at least one validity test;
c) capturing at least one image of at least a part of the test element by using the camera, the at least one part of the test element comprising at least one part of the test field; and
d) determining the concentration of the analyte in the bodily fluid from the image.

Therein, at least one of steps c) and d) is performed by taking into account the result of the validity test in step b), wherein the method comprises retrieving an image stream by using the camera, wherein step c) comprises capturing one or more still images separately from the image stream by using a still image mode of the camera of the mobile device, wherein the at least one still image provides enhanced spatial resolution and/or higher dynamics compared to the images from the image stream, wherein at least a part of the images of the image stream are flagged with at least one item of information regarding whether the image was retrieved at a point in time for which the validity test determines the angular orientation of the mobile device relative to the test element to be valid, wherein the capturing of the still image is initiated automatically in case an image of the image stream was flagged with an item of information indicating a valid image at a point in time for which the image of the image stream was flagged as being valid, and wherein step d) is performed at least partially on the at least one still image.

The term "analyte" as used herein is a broad term and is to be given its ordinary and customary meaning to a person of ordinary skill in the art and is not to be limited to a special or customized meaning. The term specifically may refer, without limitation, to arbitrary chemical, biochemical or biological substance, component or compound, such as a molecule, e.g. glucose, triglycerides, lactate or cholesterol.

The term "determining a concentration of an analyte", which may also be referred to as an analytical measurement or determination of an analyte concentration, as used herein is a broad term and is to be given its ordinary and customary meaning to a person of ordinary skill in the art and is not to be limited to a special or customized meaning. The term may specifically refer, without limitation, to a qualitative and/or quantitative determination of at least one analyte in a sample. The result of the analytical measurement, as an example, may be the concentration of the analyte and/or the presence or absence of the analyte to be determined.

The term "bodily fluid" as used herein is a broad term and is to be given its ordinary and customary meaning to a person of ordinary skill in the art and is not to be limited to a special or customized meaning. The term may specifically refer, without limitation, to a liquid sample comprising at least one bodily fluid, such as blood, interstitial fluid, urine, saliva or the like.

The term "mobile device" as used herein is a broad term and is to be given its ordinary and customary meaning to a person of ordinary skill in the art and is not to be limited to a special or customized meaning. The term may specifically refer, without limitation, to a mobile electronics device, more specifically to a mobile communication device comprising at least one processor. The mobile device may specifically be or may comprise one or more of a cell phone or a smartphone. Additionally or alternatively the mobile device may also refer to a tablet computer or any other type of portable computer having at least one camera. The mobile device, besides the at least one camera and, further, besides the at least one optional processor as outlined in further detail below, may further comprise at least one illumination source which may be used for illuminating the test element or a part thereof. Thus, as an example, the mobile device may comprise at least one light emitting diode.

The term "camera" as used herein is a broad term and is to be given its ordinary and customary meaning to a person of ordinary skill in the art and is not to be limited to a special or customized meaning. The term may specifically refer, without limitation, to a device configured for recording spatially resolved optical data, such as one or more images. The camera may specifically comprise one or more imaging devices, such as camera chips or imaging chips, e.g. CCD and/or CMOS chips. The camera, in particular the imaging device, may comprise a one-dimensional or two-dimensional array of image sensors, such as pixels. As an example, the camera may comprise at least 10 pixels in at least one dimension, such as at least 10 pixels in each dimension. It shall be noted, however, that other cameras are also feasible. The invention shall specifically be applicable to cameras as usually used in mobile applications such as notebook computers, tablets or, specifically, cell phones such as smart phones. Thus, specifically, the camera may be part of a mobile device which, besides the at least one camera, comprises one or more data processing devices such as one or more data processors. Other cameras, however, are feasible. The camera, besides at least one camera chip or imaging chip, may comprise further elements, such as one or more optical elements, e.g. one or more lenses. As an example, the camera may be a fix-focus camera, having at least one lens, which is fixedly adjusted with respect to the camera. Alternatively, however, the camera may also comprise one or more variable lenses which may be adjusted, automatically or manually.

The term "test element" as used herein is a broad term and is to be given its ordinary and customary meaning to a person of ordinary skill in the art and is not to be limited to a special or customized meaning. The term may specifically refer, without limitation, to an arbitrary element or device configured for detecting the analyte or determining the concentration of the analyte in a liquid sample, such as in the bodily fluid, specifically in a sample of the bodily fluid. The test element specifically may be or may comprise a test strip, such as an optical test strip, specifically a test strip having a polymer substrate such as a polyester or the like, e.g Melinex.

The term "test field" as used herein is a broad term and is to be given its ordinary and customary meaning to a person of ordinary skill in the art and is not to be limited to a special or customized meaning. The term may specifically refer, without limitation, to a coherent amount of at least one test chemical, such as to an area, e.g. an area of round, polygonal or rectangular shape, having one or more layers of material, with at least one layer of the test field having the test chemical comprised therein. Other layers may be present in the test field, e.g. providing specific optical properties such as reflective properties, providing spreading properties for spreading the sample or providing separation properties such as for separating off particulate components of the sample, such as cellular components.

The test element, specifically the test strip and, more specifically, the optical test strip, may comprise at least one substrate, such as at least one carrier, with the at least one test field applied thereto or integrated therein. As an example, the at least one carrier may be strip-shaped. These test strips are generally widely in use and available. One test strip may carry a single test field or a plurality of test fields having identical or different test chemicals comprised therein. The optical test strip, in particular the test field comprising the test chemical, may specifically undergo a detection reaction, particularly a coloration reaction, in the presence of the at least one analyte, specifically a coloration reaction, wherein the color formation may be related, e.g. proportional to, the concentration of the analyte. Since the presence, the absence and/or the concentration of the analyte may be detectable by the detection reaction, the detection reaction may also be referred to as analyte detection reaction. Some basic principles on test elements and reagents that may also be used within the scope of the present invention are described e.g. in J. Hönes et al.: Diabetes Technology and Therapeutics, Vol. 10, Supplement 1, 2008, pp. 10-26.

The term "angular orientation" as used herein and as specifically used in step a) is a broad term and is to be given its ordinary and customary meaning to a person of ordinary skill in the art and is not to be limited to a special or customized meaning. The term may specifically refer, without limitation, to an orientation of an object in space, such as an orientation determined by one, two, three or more angular coordinates in a coordinate system. As an example, the angular orientation may comprise information on at least one angle between an axis determined by the orientation of the mobile device, such as an optical axis of the camera, and an axis of the test element, such as an axis oriented normally to one or more of the test element or the test field. Other ways of determining the angular orientation are feasible.

The term "sensor device" as used herein is a broad term and is to be given its ordinary and customary meaning to a person of ordinary skill in the art and is not to be limited to a special or customized meaning. The term may specifically refer, without limitation, to an arbitrary device or combination of elements configured for determining at least one measurable variable and/or at least one measurable property of an object. As an example, the at least one sensor device of the mobile device may comprise at least one of an angular sensor, an orientation sensor, a magnetic field sensor, an acceleration sensor or a gyroscopic sensor. The at least one sensor device specifically may be configured for generating at least one sensor signal, more specifically at least one electronic sensor signal, which directly or indirectly, e.g. after electronic preprocessing, may be used as sensor data. Thus, the sensor data directly or indirectly may represent the at least one sensor signal indicative of the at least one measurable variable and/or measurable property determined by the sensor device. The sensor data may be or may comprise one or more of digital data or analogue data. Since the devices of this kind are generally implemented in many mobile devices, such as in many smart phones and/or portable computers such as tablet computers or notebooks.

The determining of the angular orientation by using the sensor data of the at least one sensor device may take place in various ways, as will be explained in further detail below. Thus, as an example, firstly, the orientation of the at least one test element may be determined, such as by assuming or measuring an orientation of a support on which the at least one test element rests, such as by using the at least one sensor device off the mobile device. Secondly, before or after the step described before, the orientation of the at least one mobile device may be determined by using the at least one sensor device. By comparing the orientation of the test element and the orientation of the mobile device, the orientation of the mobile device relative to the test element may be determined, such as mathematically. The step of determining the angular orientation of the mobile device relative to the test element, as an example, may be performed or supported by using at least one processor of the at least one mobile device. The determining of the angular orientation specifically may be fully or partially software-implemented in the processor.

The term "validity test" as used herein and as specifically used in step b) is a broad term and is to be given its ordinary and customary meaning to a person of ordinary skill in the art and is not to be limited to a special or customized meaning. The term may specifically refer, without limitation, to a process of determining whether one or more predetermined or determinable validity conditions are fulfilled. Thus, the subjecting of the angular orientation of the mobile device relative to the test element to the at least one validity test specifically may comprise determining if the angular orientation fulfills one or more predetermined or determinable validity conditions. As an example, the validity test may comprise checking whether the angular orientation is within at least one predetermined range, and/or may comprise comparing the angular orientation with one or more predetermined or determinable thresholds or limits. As will be outlined in further detail below, the validity test specifically may comprise comparing the angular deviation between an axis of the test element, such as an axis perpendicular to the test field of the test element, and an axis of the mobile device, such as an optical axis of the camera, with at least one tolerance threshold. As an example, ideally the deviation may be zero, whereas a predetermined tolerance may be given. Other ways of performing the validity test are also feasible. Again, the validity test specifically may be performed fully or partially by using at least one processor of the mobile device and/or may be performed fully or partially software-implemented, such as by software implemented in the processor.

Additionally or alternatively, the validity test may be or may comprise checking whether the angular orientation of the mobile device relative to the test element is within a predetermined range, such as within at least one tolerance threshold, designated to at least one coding function of a set of coding functions. The coding functions, specifically the set of coding functions, may be predetermined and may, for example, be stored in a data base or memory, such as in a memory of the mobile device.

The term "coding function" as used herein and as specifically used in step c) is a broad term and is to be given its ordinary and customary meaning to a person of ordinary skill in the art and is not to be limited to a special or customized meaning. The term may specifically refer, without limitation, to an arbitrary transformation algorithm for transforming a color or intensity of a test field from an image, specifically a color or intensity of a test field wetted by a sample to be analyzed, into a corresponding concentration of the analyte in the sample. For this purpose, the coding function, as an example, may comprise one or more of: an analytical function; a matrix algorithm or operation; a curve, such as one or more one-dimensional, two-dimensional, three-dimensional or four-dimensional curves; and a table, such as a lookup table. In particular, one coding function may be or may comprise information on the concentration of the analyte for one specific color or intensity and one angular orientation of the mobile device relative to the test element.

The set of coding functions may specifically comprise a plurality of coding functions, such as a plurality of coding functions for various colors or intensities and various angular orientations. In particular, the set of coding functions may comprise a plurality of subsets of coding functions, wherein each subset of coding functions may comprise a plurality of coding functions for various colors or intensities for one specific angular orientation. Thus, in the set of coding functions, the coding functions may be grouped or sorted according to their designated angular orientation.

When subjecting the angular orientation of the mobile device relative to the test element to the at least one validity test, the validity test may, for example, comprise checking whether in the set of coding functions there exists a coding function for which this angular orientation is within a predetermined threshold limit of the coding function's designated angular orientation.

The term "image" as used herein and as specifically used in step c) is a broad term and is to be given its ordinary and customary meaning to a person of ordinary skill in the art and is not to be limited to a special or customized meaning. The term may specifically refer, without limitation, to a set of spatially resolved optical data. The set of spatially resolved optical data may specifically comprise optical information on a region of an object. The image may also be a partial image of a larger image, e.g. a subset of spatially resolved optical data of a larger set of spatially resolved optical data. Thus, the image of an object may be sub-divided into a plurality of two or more partial images which, each by itself, may be considered as an image.

The at least one image captured in step c) may be an image of the entire test element or of a part thereof. The image at least comprises an image of the entire test field or of a part thereof. Consequently, at least a part of the test field is visible in the at least one image. Additionally, other parts of the test element may be visible in the at least one image, such as at least one part of a substrate of the test elements, specifically of the test strip, such as at least one white part of the test strip. The at least one additional part of the test element visible in the at least one image may be used for evaluation purposes, too.

As outlined above, in step d), the concentration of the analyte in the bodily fluid is determined from the image. As an example, the step may comprise deriving at least one item of information from the image, such as at least one item of color information, e.g. at least one item of color information indicative for a detection reaction taking place in the test field. As an example, at least one item of color information may be derived which is indicative for a reflectance of the test field which changes with the concentration of the analyte in the bodily fluid. These ways of determining the concentration of the analyte in the bodily fluid from at least one item of color information derived from an image of generally known to the skilled person.

As outlined above, at least one of steps c) and d) is performed by taking into account the result of the validity test performed in step b). Thus, several possibilities exist, as will be outlined in further detail below. Thus, firstly, the capturing of the at least one image in step c) may be made dependent on the result of the validity test. As an example, the image may be captured and only in case the validity test returns a specific result, such as the angular orientation of the mobile device relative to the test element being within a predetermined range of tolerance. Additionally or alternatively, secondly, the determining of the concentration of the analyte in the bodily fluid from the image may be made dependent on the result of the validity test. Thus, as an example, an image captured may be used for the determining of the concentration only if the validity test returns a specific result, such as the angular orientation of the mobile device relative to the test element when capturing the image being within a predetermined range of tolerance.

As outlined above, the validity test in step b) may be performed in various ways. Specifically, the validity test in step b) may comprise comparing the at least one angular orientation of the mobile device relative to the test element, such as one or more of angular values indicative for the angular orientation, with at least one target orientation. The target orientation, for example, may be a specific angle or combination of angles indicative for the angular orientation of the mobile device relative to the test element. As an example, as outlined above, the target orientation may be an orientation in which an axis perpendicular to the test element, the test strip or the test field is oriented parallel to an optical axis of the camera of the mobile device. The target orientation, as an example, may be predetermined or predefined.

The target orientation may be an orientation in which an axis perpendicular to the test element, the test strip or the test field is oriented at a predetermined and/or predefined angle with respect to the optical axis of the camera of the mobile device. In particular, the target orientation may be an orientation in which the axis perpendicular to the test element, the test strip or the test field in oriented in a nonparallel fashion to the optical axis of the camera of the mobile device. Specifically, the target orientation, such as an angle θ between the test strip or the test field and the mobile device, e.g. the optical axis of the mobile device, may for example be θ ≠ 0°. As an example, the target orientation may be 0° < θ ≤ 50°, specifically 1° ≤ θ ≤ 45°, more specifically 5° ≤ θ ≤ 40°.

The validity test may return at least one test result. The test result, as an example, may be or may comprise a Boolean result, such as "true"/"false" or "valid" and "invalid". Specifically, the validity test may determine the angular orientation of the mobile device relative to the test element
- to be valid in case the angular orientation deviates from the target orientation by no more than at least one predetermined angular tolerance; or
- to be invalid in case the angular orientation deviates from the target orientation by more than the at least one predetermined angular tolerance.

The angular tolerance, as an example, may indicate a maximum deviation of the angular orientation from a parallel orientation between test field and the camera, i.e. between the optical axis of the camera and an axis perpendicular to the test field and/or test element, such as a maximum deviation of no more than 20°, specifically of no more than 10°, more specifically of no more than 5°. When taking into account the result of the validity test when performing one or both of steps c) or d), as an example, the Boolean variable may be taken into account at the result of the validity, such as by enabling and/or triggering performing step c) only in case the test result has a specified value, such as "valid", and/or by determining the concentration in step d) only for one or more images for which the test result has a specified value, such as "valid".

As outlined above, the result of the validity test may be used for one or more of blocking, enabling our triggering the capturing of the at least one image. Additionally or alternatively, however, the result of the validity test may be used when determining the concentration of the analyte in the body fluid, such as by evaluating images only which are captured in a condition under which the validity test returns a specified value, such as "valid".

Thus, specifically, the method may comprise monitoring the angular orientation of the mobile device relative to the test element. The monitoring, as an example, may be performed continuously, in regular time interval or at specified points in time. The monitoring may comprise a repeated performing of the validity test. The test result of the validity test may be recorded. The method may comprise blocking the capturing of the at least one image in case the validity test determines the angular orientation of the mobile device relative to the test element to be invalid, such as being out of a tolerance range. The method may further comprise unblocking the capturing of the at least one image in case the validity test determines the angular orientation of the mobile device relative to the test element to be valid. Additionally or alternatively, the method may comprise automatically initiating, such as automatically triggering, the capturing of the at least one image in case the validity test determines the angular orientation of the mobile device relative to the test element to be valid.

The term "capturing" as used herein and as specifically used in step c) is a broad term and is to be given its ordinary and customary meaning to a person of ordinary skill in the art and is not to be limited to a special or customized meaning. The term may specifically refer, without limitation, to the recording of the at least one image, such as the recording of image data of the at least one image. The image data specifically may be stored in at least one data storage device, such as in at least one data storage device of the mobile device.

In addition, the method comprises retrieving an image stream, such as a continuous image stream, by using the camera. These image streams are generally retrieved when viewing a live scene e.g. via a display of the mobile device, such as the smart phone. Thus, the image stream may generally comprise a series of images which may be recorded only temporarily, such as for the purpose of displaying the current image of the stream on the display, such as in real time. The capturing of the at least one image in step c) may comprise selecting the at least one image from the image stream. The capturing in step c) may further comprise storing the selected image in at least one data storage device.

The selection of the at least one image from the image stream is initiated automatically, e.g. by the depending on the result of the validity test in step b). Thus, as an example, as soon as the validity test returns the angular orientation of the mobile device relative to the test element being within a predetermined tolerance range, one or more corresponding images may be selected from the registry, either in real time or subsequently, and may be used as the one or more captured images.

The at least one image specifically may be selected from the image stream in case the image was retrieved at a point in time for which the validity test determines the angular orientation of the mobile device relative to the test element to be valid. Additionally or alternatively, at least a part of the images of the image stream is flagged with at least one item of information regarding whether the image was retrieved at a point in time for which the validity test determines the angular orientation of the mobile device relative to the test element to be valid. Thus, the result of the validity test may be used e.g. for the selection of the at least one image from the image stream and/or is also used for flagging all or at least some of the images of the image stream, for further processing. Thus, as an example, the image stream may be stored at least partially, including e.g. images for which the validity test returns the result of the angular orientation being valid or within a predetermined tolerance range, wherein, in the processing of the image stream, the one or more images of the image stream flagged as valid may be used as the captured images, for determining the concentration of the analyte in the bodily fluid. Thus, generally, step d) may be performed at least partially on the basis of at least one image flagged with the item of information indicating that the image was retrieved at a point in time for which the validity test determines the angular orientation of the mobile device relative to the test element to be valid.

The method, in step c), comprises capturing one or more still images by using the camera in case at least one image of the image stream was flagged with an item of information indicating a valid image or indicating the at least one image being valid. Thus, the still image is not selected from the images of the image stream but is captured separately by using a still image mode of the camera of the mobile device. The capturing of the still image is initiated automatically in case an image of the image stream was flagged with an item of information indicating a valid image. The still image is captured at a point in time for which the image of the image stream was flagged as being valid. Further, step d) isper-formed at least partially on the at least one still image.

The resolution of an image of the image stream may generally be lower than the spatial resolution of a specific still image captured by the camera, wherein the spatial resolution may refer to the resolution of n x m pixel of the image. Further, regarding the signal dynamics, the image stream may commonly only provide images with a 8-bit resolution for each of the R, G, B color channels, while captured still images may also provide higher resolution, for example images with a resolution of at least 10-bit. Additionally, the time interval between two captured images may be higher for captured still images than for images of the image stream. In general, optical measurements of a color formation reaction of a test strip may use images selected from an image stream for evaluating the test field at the right time.

In particular, low-end mobile devices and/or older mobile device may often provide images from the image stream having a low pixel resolution, for example a VGA resolution of 640 x 480 pixels. Thus, the number of pixels per reference field, specifically per gray color reference field, may generally be small. Further, the image stream may have low signal dynamics in each of the R, G, B color channels and, thus, may only provide limited possibility of mapping the measured signal of the color of the test field to the available measurement values. For example, the measurement range of the glucose concentration may typically be within the range of 0 to 600 mg/dl. In case the captured image may have an 8-bit dynamics with a lower limit of 50 counts and an upper limit of 200 counts, the resulting measurement range may be 4 mg/dl per count of the measurement signal.

Thus, as outlined above, the method, in step c), comprises capturing one or more still images and at least one image stream by using the camera. The image stream is used for user guidance, for validity testing and, optionally, for testing the kinetics of the images. One or more still images are captured separately from the image stream in case an image of the image stream was flagged as valid. The at least one still image provides enhanced spatial resolution and/or higher dynamics compared to the images from the images stream. The still image may provide a higher number of pixels for each reference field due to the higher spatial resolution and, thus, a higher statistical significance with regard to the item of color information of the test field derived from the image. Further, the still image may provide an enhanced measurement performance due to the higher dynamics. Thus, this approach may improve the compatibility with a higher number of different mobile devices and may also improve the measurement performance of the color of the test field.

As outlined above, various ways exist for determining the angular orientation of the mobile device relative to the test element. Thus, as an example, step a) at least partially may be performed under the assumption that the test element is oriented in a predetermined orientation. This assumption often is reasonable in case the test element is placed on a table or another support having a predetermined angular orientation. As an example, the predetermined orientation may be a horizontal orientation, as often is the case for a table or another support used in daily practice.

Additionally or alternatively, as also mentioned above, the angular orientation of a support on which the test element rests during the capturing of the image may be checked by using the mobile device, e.g. before or after performing step c). Thus, as an example, the method may further comprise determining an angular orientation of the test element by placing the mobile device on a support surface and determining an angular orientation of the support surface. This determination of the angular orientation of the support surface may also be performed by using sensor data of the at least one sensor device integrated into the mobile device. Again, this step of determining the angular orientation of the support surface, e.g. in a coordinate system of the mobile device, may be performed at least partially automatically, e.g. user initiated or automatically without user interaction, such as by assuming that the user at least once during the method places the mobile phone on the support surface, e.g. during an application of a sample of the bodily fluid to the test element, specifically to the test field. The step of determining the angular orientation of the support surface may, again, be fully or partially software implemented. In step a), the test element may be placed on the support surface. Thus, since the angular orientation of the support surface is known, and, consequently, the angular orientation of the test element is known, and, further, since the orientation of the mobile device during capturing the at least one image is also known or may be determined by using the sensor data of the at least one sensor device, the relative angular orientation of the mobile device relative to the test element may be determined by comparing the angular orientation of the test element and the angular orientation of the mobile device.

As outlined above, the method may further comprise applying at least one sample of the bodily fluid to the test element. The application of the sample of the bodily fluid to the test element specifically may be performed at least once before performing step c). Still, however, step c) may also be performed repeatedly, wherein step c) may be performed at least once without having applied the at least one sample of the bodily fluid to the test element and wherein at least one iteration of step c) is performed after application of the sample of the bodily fluid to the test element. Thereby, at least one dry image, without having sample applied to the test field may be captured, and, further, at least one wetted image, having sample applied to the test field, may be captured in step c). In step d), at least the wetted image may be used, wherein, additionally, however, the dry image may also be used, e.g. for reference purposes. In both cases, as outlined above, besides at least one part of the test field visible in the image, optionally at least one further part of the test element and/or of a different part, such as of a color reference card, may be visible and may be used for evaluation purposes, such as a white field. Thus, as an example, both for the at least one wetted image and, optionally, for the optional at least one dry image a relative value of at least one item of color information relative to the reference part, such as the white field, may be derived. Thus, as an example, the concentration of the analyte may be determined by comparing the relative value of the at least one item of color information for the test field to the at least one item of color information for the reference field, such as the white field, of the at least one wetted image and the at least one dry image. Alternatively, however, only the relative value of the at least one item of color information for the test field to the at least one item of color information of the reference field for the wetted image may be used for determining the analyte concentration. Again, alternatively, only the at least one item of color information for the test field of the wetted image may be used for determining the analyte concentration. The at least one optional reference field may be part of the test element or, as an example, may be part of a reference card or a reference element. The reference card or the reference element having the at least one optional reference field, such as at least one color reference field, may be placed in the field of view of the camera during capturing the image in step c) or, additionally or alternatively, may be part of an additional reference image captured independently.

Thus, generally, step c) may further comprise capturing at least one dry reference image, also simply referred to as a dry image, of the at least one part of the test element before applying the bodily fluid to the test element. Step c) may then further comprise capturing at least one measurement image, also referred to as a wetted image, of the at least one part of the test element after applying the bodily fluid to the test element. In step d), both the dry image and the measurement image may be taken into account for determining the concentration of the analyte in the bodily fluid. Therein, generally, steps a), b) and c) may be performed at least once for capturing the dry image and at least once for capturing the measurement image. Thus, the angular orientation of the mobile device relative to the test element, the validity test and the capturing of the image may be performed both for the dry state and for the wetted state.

Step c) may further comprise capturing at least one color reference image, also simply referred to as a reference image, of at least one color reference, wherein, in step d), the color reference image is taken into account for determining the concentration of the analyte in the bodily fluid. Additionally or alternatively, however, as outlined above, the at least one image captured in step c), in the wetted state and optionally also in the dry state, may also comprise the color reference, e.g. as a part of the image, e.g. as a white part of the test strip visible in the image.

The method may generally comprise providing user guidance for guiding a user towards a target orientation of the mobile device relative to the test element, e.g. the same target orientation as optionally used in the validity test. The user guidance specifically may comprise visual guidance on a display of the mobile device. User guidance of this type may comprise any type of visual guidance indicating a target state of angular orientation and a current state of angular orientation, in order to allow for the user to bring the mobile device into the target orientation, by bringing the indicator for the current orientation as close as possible to the indicator for the target orientation or the like. As an example, circles may be used for the target orientation and the current orientation, or other visual indicators as generally known e.g. for leveling devices.

The method according to the present invention specifically may be performed such that the method does not imply a mathematical correction of the at least one image for angular misalignment. Specifically, the method may be performed such that no information on the angular orientation of the mobile device relative to the test element is derived from the at least one image. Thus, the method generally may avoid an analysis of geometrical image data for determining the angular orientation. By avoiding the analysis of geometrical image data for determining the angular orientation of the mobile device relative to the test element, e.g. by using sensor data of the mobile device only, the method may be rendered more efficient than methods based on image analysis for determining the angular orientation.

In a further aspect of the present invention, a mobile device is proposed, having a camera. The mobile device is configured for determining a concentration of at least one analyte in a bodily fluid by using the test element, the mobile device being configured to perform the method according to claims 1, 2, 3, 5, 7 and 8.

For possible embodiments or definitions of the mobile device, thus, reference may be made to the description of the method.

As outlined above, the mobile device specifically may comprise at least one processor. The term "processor" as used herein is a broad term and is to be given its ordinary and customary meaning to a person of ordinary skill in the art and is not to be limited to a special or customized meaning. The term specifically may refer, without limitation, to a data processing device or to a combination of data processing devices capable of executing a program or a series of instructions. As an example, the processor may comprise a single integrated processor circuit, such as a single processor chip. Additionally or alternatively, the processor may comprise a network of processor chips. The processor may further, additionally or alternatively, comprise at least one application-specific integrated circuit and/or at least one field-programmable gate array. Further additionally or alternatively, the processor may fully or partially be integrated with other hardware components, such as by using a virtual machine running on a larger processor or a computer device.

When referring to the method steps of the method described herein, all of these method steps may fully or partially be supported by software executed by the at least one processor of the mobile device. Thus, the sensor data of the at least one sensor device may be provided to the processor for fully or partially performing step a). Further, the validity test in step b) may fully or partially be software-implemented, executed by the processor. Further, the capturing of the at least one image in step c) by using the camera may be one or more of initiated, controlled or triggered by software executed by the processor. Further, the determining of the concentration of the analyte in the bodily fluid from the at least one image in step d) may be fully or partially performed by software executed by the processor.

The mobile device may further be configured for prompting the user to apply a sample of the bodily fluid to the test element. This prompting, as an example, may take place e.g. at least once before performing step c). As outlined above, however, step c) may be performed repeatedly, wherein, as an example, step c) may be performed at least once before applying the sample of the bodily fluid to the test element and at least once after applying the sample of the bodily fluid to the test element. Thus, as an example, the mobile device may further be configured for prompting the user to at least once perform step c) without having the sample applied to the test element and, subsequently, may be configured for then prompting the user to apply the sample to test element. The prompting may take place in various ways generally known to the skilled person, such as by displaying a corresponding message or instructions on a display of the mobile device and/or by providing audible instructions.

The mobile device, as outlined above, specifically may comprise at least one mobile electronic device, more specifically a mobile communication device, more specifically one or more of a cell phone, a smart phone, a portable computer.

In a further aspect of the present invention, a kit is proposed, the kit comprising at least one mobile device according to claim 9, and, further, at least one test element having at least one test field. The term "kit" as used herein is a broad term and is to be given its ordinary and customary meaning to a person of ordinary skill in the art and is not to be limited to a special or customized meaning. The term specifically may refer, without limitation, to a combination of at least two items which might as an example, may be supplied conjointly in a package, which may interact in order to fulfill at least one common purpose. The kit may further comprise at least one reference card, the reference card having at least one reference color field.

In a further aspect of the present invention, a computer program is proposed, the computer program comprising instructions which, when the program is executed by a mobile device having a camera and, further, optionally, at least one processor, cause the mobile device to carry out the steps of the method according to the present invention, such as according to any one of the embodiments disclosed above and/or according to any one of the embodiments disclosed in further detail below.

In still a further aspect of the present invention, a computer-readable storage medium is proposed, comprising instructions which, when executed by a mobile device according to claim 9, cause the mobile device to carry out the steps of the method according claims 1, 2, 3, 5, 7 and 8.

Specifically, the computer program may be stored on a computer-readable data carrier and/or on a computer-readable storage medium. As used herein, the terms "computer-readable data carrier" and "computer-readable storage medium" specifically may refer to non-transitory data storage means, such as a hardware storage medium having stored thereon computer-executable instructions. The computer-readable data carrier or storage medium specifically may be or may comprise a storage medium such as a random-access memory (RAM) and/or a read-only memory (ROM).

Thus, specifically, one, more than one or even all of method steps a) to d) as indicated above may be performed by using a computer or a computer network, preferably by using a computer program.

The method and the devices according to the present invention provide a large number of advantages over known methods and devices of similar kind. Thus, firstly, the present invention specifically may take account of the fact that, when using a camera based evaluation of optical test strips, both the illumination angle, i.e. the angle between an axis perpendicular to a test strip and the direction of illumination, and the observation angle, i.e. the angle between the axis perpendicular to the test strip and the direction of observation of the test field, may have a significant impact on the result of the measurement, specifically on the determined analyte concentration. Specifically, the physical effect of scattering of light, which may strongly be dependent on the material of the test strip, the test field or the reference color field, as well as on the angle of incidence may be taken into account for the measurement. By taking into account the angular orientation, these physical effects of uncertainty may be eliminated or, at least, reduced.

Applying the present invention, thus, significant uncertainties of the measurement, which may sum up to more than 100% deviation due to the angular uncertainties and the uncertainties of the scattering of light, may be eliminated or at least reduced. Thus, generally, measurement errors due to varying angular orientation of the mobile device, such as the smart phone, relative to the test element may be eliminated or at least reduced.

Therein, several measurement setups and methods for determining the analyte concentration may be used, implementing the ideas of the present invention. Thus, firstly, as outlined above, a relative value of at least one item of color information of the test field compared to at least one item of color information of at least one reference field may be derived from the at least one image. As an example, a quotient of the at least one item of color information of the at least one test field and the at least one item of color information of the at least one reference field may be determined. The relative value may be determined both for the dry state and for the wetted state, as outlined above. The latter generally may be referred to as a double relative measurement. This double relative measurement, generally, provides for accurate measurement results, since color variations of manufacturing of the test elements may at least partially be eliminated. Still, however, when using mobile devices for the measurement, these double relative measurements generally are specifically prone to angular misalignments, since measurements are taken both for the dry state and for the wetted state, including the doubled risk of varying angular orientations. By using the present invention and by taking into account the angular orientation, e.g. both for the measurement in the dry state and the measurement in the wetted state, angular misorienta-tions may be taken into account, thereby significantly increasing the measurement accuracy.

Additionally or alternatively, a simple relative measurement may be performed. Therein, again, a relative value of at least one item of color information of the test field compared to at least one item of color information of at least one reference field may be derived from the at least one image, for the wetted state, without performing a relative measurement in the dry state. Again, as an example, a quotient of the at least one item of color information of the at least one test field and the at least one item of color information of the at least one reference field may be determined and may be used for determining the analyte concentration. Again, by using the present invention, the impact of angular misalignment between the mobile device and the at least one test element on the measurement result, specifically on the concentration of the analyte in the body fluid, may be eliminated or, at least, reduced.

The method according to the present invention specifically may make use of at least one sensor device which is typically implemented in standardized mobile devices, such as in smart phones, tablet computers or the like. As an example, many mobile devices, such as smart phones or tablet computers, comprise at least one of an angular sensor, an orientation sensor, a magnetic field sensor, an acceleration sensor or a gyroscopic sensor. Sensor data from one or more of these sensor devices may be used for determining the angular orientation. According to the present invention, as an example, the information on the angular orientation may be used for one or more of:
- capturing and evaluating at least one image only in case the validity test determines the angular orientation to be valid;
- when recording an image stream, selecting images from the image stream which were taken under angular conditions for which the validity test determines the angular orientation to be valid;
- recording an image stream, wherein images for which the validity test determines the angular orientation to be valid are marked in a specific way, i.e. are "flagged", wherein, for determining the concentration of the analyte in the body fluid, only one or more of these marked images are used.

Other possibilities for making use of the information on the angular orientation may be given. Further, combinations of the named options are also possible, depending on the specific application.

The target orientation may be an orientation in which the mobile device is parallel to the test element, such as to the test strip, and/or to the reference color field or reference color card. Therein, a parallel orientation generally may refer to the case in which an axis perpendicular to the test strip, such as to the test field, and/or an axis perpendicular to the reference color field, such as to the reference color card, is parallel to an optical axis of the camera. Alternatively, however, the target orientation may also deviate from a parallel orientation. Thus, as an example, a target orientation having an inclination angle of e.g. 5°, 10° or the like may be given, such as predetermined. Further, for taking an image of at least one reference color field, the target orientation may be different to the target orientation for taking an image of the at least one test field. Further, the angular tolerances predetermined for tolerable deviations from the target orientation may, as an example, be in the range of +/- 20°, +/- 10°, +/- 5° or +/- 2°. Other tolerance ranges may be given. Thus, generally, the method may be adapted e.g. to illumination conditions, measurement conditions or materials used for the measurement.

The target orientation, such as an angle θ between the optical axis of the mobile device and the axis perpendicular to the test strip and/or to the reference color field and/or to the reference color card, may be θ ≠ 0°. Specifically, the target orientation, for example the angle θ between the optical axis of the mobile device and the axis perpendicular to the reference color card or the reference color field, may for example be 0° < θ ≤ 50°, specifically 1° ≤ θ ≤ 45°, more specifically 5° ≤ θ ≤ 40°. In particular, as an example, the target orientation between the mobile device and the reference color card, specifically between the mobile device and the reference color field, may be θ = 40°.

In particular, the target orientation, e.g. the angle θ, may be the same for the test strip and for the reference color field and/or the reference color card. As an alternative however, the target orientation, i.e. the angle θ, may be different for the test strip than for the reference color field and/or the reference color card. Specifically, the target orientation for the test strip, such as the angle θ between the optical axis of the mobile device and the axis perpendicular to the test strip, may differ from the target orientation for the reference color field and/or the reference color card, i.e. the angle θ between the optical axis of the mobile device and the axis perpendicular to the reference color field and/or to the reference color card. As an example, the target orientation for the test strip, specifically the angle θ between the optical axis of the mobile device and the test strip, may be θ = 1° or θ = 2° with a respective tolerable deviation of +/- 20°, +/- 10°, +/- 5° or +/- 2°, while the target orientation for the reference color field and/or reference color card, specifically the angle θ between the optical axis of the mobile device and the reference color field and/or card, may be θ = 40°, θ = 50° or θ = 60° with a respective tolerable deviation of +/- 20°, +/- 10°, +/-5° or +/- 2°.

The present invention may be applied in various ways and may be adapted to the circumstances of the measurement. Specifically, for determining the angular orientation of the mobile device relative to the test element the following options, inter alia, may be used.

The angular orientation of the mobile device relative to the test element may be determined by assuming a specific orientation of the test element, such as of the test strip. As an example, it is generally reasonable to assume that the test elements, when resting on a table, is oriented in a horizontal fashion, i.e. with an axis perpendicular to the test element or test field being oriented in a vertical direction. For verifying this assumption, the method may further comprise providing user guidance, e.g. by visual and/or audible instructions, in order to have the user plays the test element on a horizontal support.

Additionally or alternatively, the angular orientation or a variation of the angular orientation may be determined for one or more of a dry state or a wetted state.

Further additionally or alternatively, as outlined above, an angular orientation of a support surface may be determined, e.g. prior or after the capturing of the image of the test field. As also outlined above, this process may also be performed automatically, e.g. by assuming that the mobile device, during the whole procedure of determining the concentration of the analyte, at least once is placed and rests on the support surface. As an example, the mobile device typically is placed on the support surface e.g. during preparing the test element, preparing a tracking device or the like. Thus, as an example, sensor data of at least one sensor of the mobile device may be monitored, in order to determine whether the mobile device rests on a support surface, wherein, when a measurement takes place e.g. in a predetermined timeframe before or after this rest, the orientation of the mobile device in the resting state is assumed to correspond to the orientation of the support surface.

Again additionally or alternatively, the angular orientation of the mobile device relative to a reference card having at least one reference color field may be determined for capturing at least one image of the at least one reference color field. Again, one or more of the above-mentioned options may be applied. Specifically, as an example, at least one marker on the reference card may be used for determining the angular orientation. As an example, at least one ArUco marker on the reference card may be used.

By using the sensor data and/or information on the angular orientation, user guidance may be provided. Thus, as an example, the user may be guided in an efficient way to establish a desired angular orientation of the mobile device relative to the test element. As an example, visual guidance markers as e.g. typically used for leveling may also be applied for user guidance.

The method according to the present invention specifically may be performed without correcting the at least one image captured in step c) for angular misalignment. Thus, no algorithms of angular correction of the image may be applied, such as corrections in which, using known information on dimensions of the test field and/or other characteristic features of the test element are used for determining angular misalignments. The angular orientation of the mobile device relative to the test element may be taken into account by using sensor data of the at least one sensor device integrated into the mobile device, only, without additional mathematical angular correction. This sensor-based procedure, as opposed to algorithmic angular correction, may significantly reduce the use of resources of the mobile device. Thus, as compared to image correction methods based on algorithms, the present invention may provide for a fast and efficient way of determining the concentration of the analyte which specifically is suitable for mobile devices having limited hardware resources.

### Short description of the Figures

Further optional features and embodiments will be disclosed in more detail in the subsequent description of embodiments, preferably in conjunction with the dependent claims. Therein, the respective optional features may be realized in an isolated fashion as well as in any arbitrary feasible combination, as the skilled person will realize. The scope of the invention is not restricted by the preferred embodiments. The embodiments are schematically depicted in the Figures. Therein, identical reference numbers in these Figures refer to identical or functionally comparable elements.

In the Figures:
- Figure 1: shows a perspective view of an embodiment of a kit and a mobile device;
- Figure 2: shows an embodiment of an image of a part of the test element captured by a camera of the mobile device;
- Figure 3: shows a flow chart of a method of determining a concentration of at least one analyte in a bodily fluid by using a mobile device and a test element;
- Figure 4: shows a top plane view of an embodiment of a mobile device; and
- Figure 5: shows an embodiment of a graph of backscattered intensity over a polar angle.

### Detailed description of the embodiments

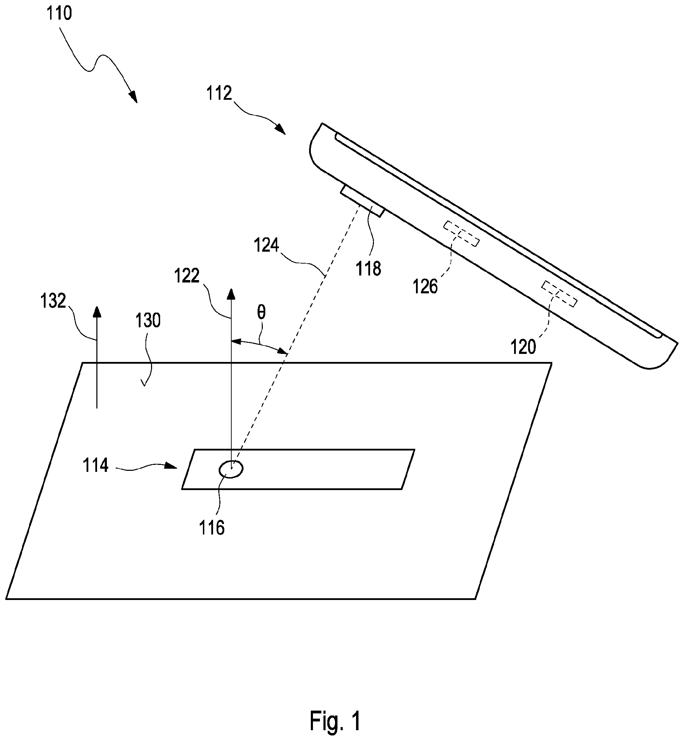

In Figure 1 a perspective view of an embodiment of a kit 110 and a mobile device 112 is shown. The kit 110 comprises the mobile device 112 and at least one test element 114 having at least one test field 116. The mobile device 112 comprises a camera 118 and is configured for determining a concentration of at least one analyte in a bodily fluid by using the test element 114. Specifically, the mobile device 112 is configured to perform the method of determining a concentration of at least one analyte in a bodily fluid. In particular, at least one sensor device 120 may be integrated into the mobile device 112. Sensor data of the at least sensor device 120 may be used for determining an angular orientation of the mobile device 112 relative to the test element 114. As an example, an angle θ between a normal direction 122 of the test element 114 and an image capturing direction 124 of the camera of the mobile device 112 may be determined. The mobile device 112 may further comprise at least one processor 126. The processor 126 may for example be configured for processing the at least one image 128 of at least a part of the test element 114 captured by using the camera 118 of the mobile device 112.

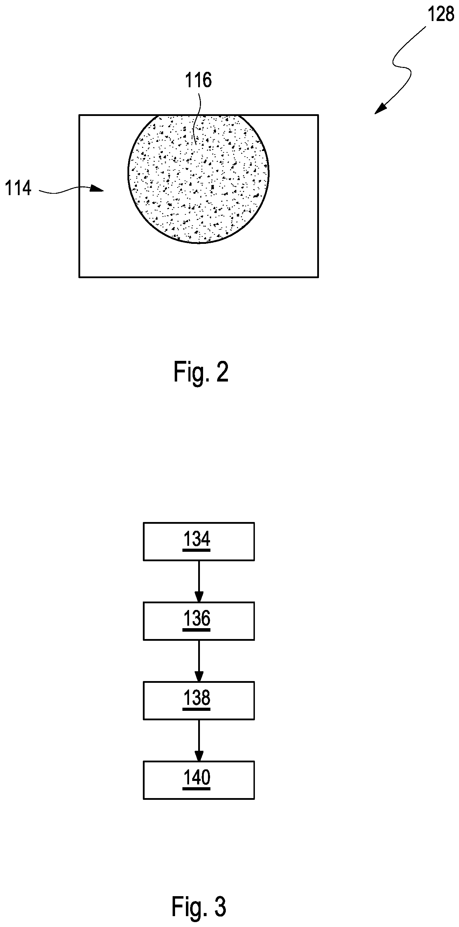

In Figure 2 an embodiment of an image 128 of a part of the test element 114 captured by using the camera 118 of the mobile device 112 is shown. The part of the test element 114 illustrated in the image 128 comprises at least one part of the test field 116 of the test element 114.

As illustrated in Figure 1, the test element 114 may be placed on a support surface 130. In particular, an angular orientation 132 of the support surface may be determined, for example by placing the mobile device 112 on the support surface 130. As an example, additionally or alternatively to the normal direction 122 of the test element 114, the angular orientation 132 of the support surface may be used for determining the angle θ.

Figure 3 shows a flow chart of a method of determining a concentration of at least one analyte in a bodily fluid by using a mobile device 112 and a test element 114. The method comprises the following steps, which may specifically be performed in the given order. Still, a different order may also be possible. It may be possible to perform two or more of the method steps fully or partially simultaneously. It may further be possible to perform one, more than one or even all of the method steps once or repeatedly. The method may comprise additional method steps which are not listed herein. The method steps of the method of determining a concentration of at least one analyte in a bodily fluid are the following:
step a) (denoted with reference number 134) determining an angular orientation of the mobile device 112 relative to the test element 114 by using sensor data of at least one sensor device 120 integrated into the mobile device 112;
step b) (denoted with reference number 136) subjecting the angular orientation of the mobile device 112 relative to the test element 114 to at least one validity test;
step c) (denoted with reference number 138) capturing at least one image 128 of at least a part of the test element 114 by using the camera 118, the at least one part of the test element 114 comprising at least one part of the field 116; and
step d) (denoted with reference number 140) determining the concentration of the analyte in the bodily fluid from the image 128;

At least one of steps c) and d) is performed by taking into account the result of the validity test in step b). In particular, the validity test in step b) may comprise comparing the angular orientation of the mobile device 112 relative to the test element 114 with at least one target orientation. Thus, the angular orientation of the mobile device 112 relative to the test element 114, as for example illustrated in Figure 1, may be subjected to the at least one validity test. Specifically, the validity test may determine the angular orientation of the mobile device 112 relative to the test element 114 to be valid or to be invalid. In particular, in the validity test, the angular orientation of the mobile device 112 relative to the test element 114 may be compared to a target orientation. The angular orientation of the mobile device 112 relative to the test element 114 may specifically be valid in case the angular orientation deviates from the target orientation by no more than at least one predetermined angular tolerance. Alternatively, the angular orientation of the mobile device 112 relative to the test element 114 may be invalid in case the angular orientation deviates from the target orientation by more than the at least one predetermined angular tolerance. As an example, in the validity test, the angle θ may be compared to an angle of a target orientation with the predetermined angular tolerance. As an example, the target orientation may be parallel to the angular orientation of the mobile device 112. Thus, the angle of the target orientation may specifically be equal to 0°. For example, the angular orientation of the mobile device 112 may be targeted to be plane parallel to the angular orientation of the test element 114. Alternatively, the target orientation may be selected to be non-parallel to the angular orientation of the mobile device 112. It may particularly be useful for the target orientation, for example, to be equal to 10°. Specifically, the predetermined angular tolerance may be +/-10°. More specifically, the predetermined angular tolerance may be +/- 5°. In particular, the predetermined angular tolerance may be +/- 2°.

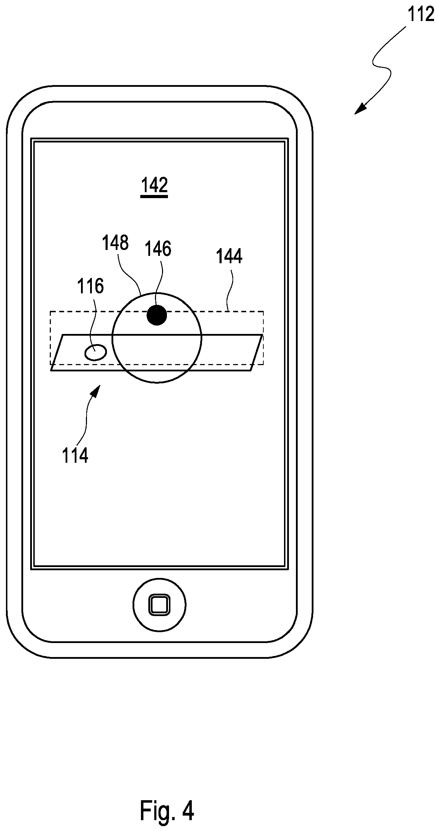

In Figure 4, a top plane view of an embodiment of a mobile device 112 is shown. The mobile device 112 may specifically be in the process of performing the method of determining a concentration of at least one analyte in a bodily fluid, as illustrated in Figure 3. The method may comprise monitoring the angular orientation of the mobile device 112 relative to the test element 114. Specifically, in case the validity test determines the angular orientation of the mobile device 112 relative to the test element 114 to be invalid, the method may further comprise blocking the capturing of the at least one image 128. However, in case the validity test determines the angular orientation of the mobile device 112 relative to the test element 114 to be valid, the method may comprise unblocking the capturing of the at least one image 128. Additionally or alternatively, the method may comprise automatically initiating the capturing of the at least one image 128 in case the validity test determines the angular orientation of the mobile device 112 relative to the test element 114 to be valid. Additionally or alternatively, the method comprises continuously retrieving an image stream by using the camera 118, wherein the capturing in step c) of the method may comprise selecting the at least one image 128 from the image stream. Specifically, the image 128 may be selected from the image stream in case the image 128 was retrieved at a point in time for which the validity test determines the angular orientation of the mobile device 112 relative to the test element 114 to be valid.

Further, the method may comprise providing user guidance for guiding a user towards a target orientation of the mobile device 112 relative to the test element 114. Specifically, the user guidance may comprise a visual guidance on a display 142 of the mobile device 112. As illustrated in Figure 4, the visual guidance on the display 142 of the mobile device 112 may for example be a virtual outline 144 of the test strip 114. Additionally or alternatively, the visual guidance on the display 142 of the mobile device 112 may for example guide the user towards the target orientation of the mobile device 112 relative to the test element 114 by visually indicating in which direction the mobile device 112 is to be moved or tilted. In particular, the user guidance may make use of a spirit level function sensor. As an example, the visual guidance may comprise a visually illustrated ball 146 within a circle 148 shown on the display 142, wherein the user may be requested to guide the ball 146 towards the center of the circle 148. Specifically, the user may be requested to guide the ball 146 towards center of the circle 148 by moving and/or tilting the mobile device 112.

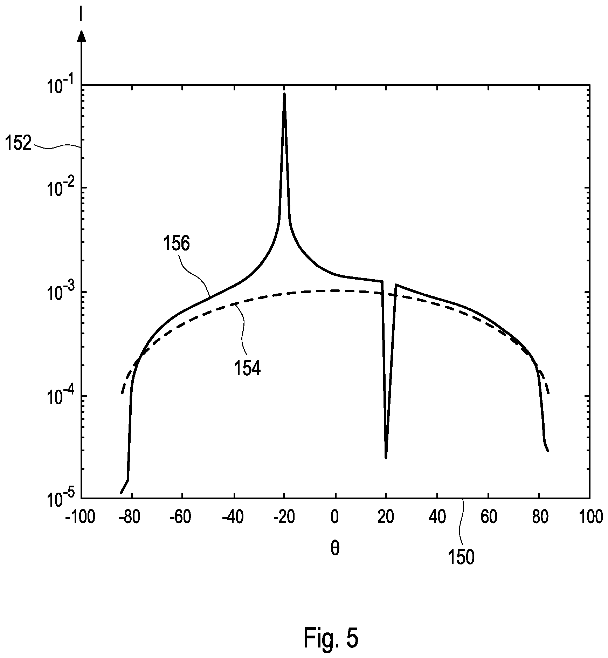

In Figure 5 an embodiment of a graph of backscattered intensity over a polar angle is illustrated. The x-axis 150 shows a polar angle, specifically the angle θ, and the y-axis 152 shows the backscattered intensity I. In the graph, the dashed line illustrates a Lambertian reflectance 154, such as a reflectance of a surface which obeys Lambert's law, wherein the intensity observed from an ideal diffusely reflecting surface is directly proportional to the cosine of the angle θ between the direction of the incident light and the surface normal. In particular, when showing a Lambertian reflectance 154, a surface may have the same radiance when viewed from any angle θ. Thus, for example, the surface may have the same brightness or luminance at θ = 20° and θ = -20°. In particular, the test field 116 of the test element 114 may for example show a Lambertian reflectance 154.

Further, in the graph, the solid line illustrates a measured reflectance 156, specifically a measured reflectance of for example the test element 114, such as for example a reflectance of a surface of the test element 114 except for the test field 116. As illustrated, the measured reflectance 156 shows a peak reflectance at -20°. In particular, the peak reflectance at -20° may have been unusual high width. A similar peak, though without the extremely high width, may be observed in the measured reflectance 156 at +20°. As an example, the peaks in the measured reflectance may interfere with a determination of the analyte in the bodily fluid from an image taken at -20° or +20°. Thus, it may be beneficial to avoid using such images for determining the analyte in the bodily fluid.

### List of reference numbers

- 110: kit
- 112: mobile device
- 114: test element
- 116: test field
- 118: camera
- 120: sensor device
- 122: normal direction of the test element
- 124: image capturing direction
- 126: processor
- 128: image
- 130: support surface
- 132: angular orientation of the support surface
- 134: step a)
- 136: step b)
- 138: step c)
- 140: step d)
- 142: display
- 144: outline of the test strip
- 146: ball
- 148: circle
- 150: x-axis
- 152: y-axis
- 154: Lambertian reflectance
- 156: measured reflectance

## Claims

1. A method of determining a concentration of at least one analyte in a bodily fluid by using a mobile device (112) and a test element (114), the mobile device (112) having a camera (118), and the test element (114) having at least one test field (116), the method carried out by the mobile device comprising
a) determining an angular orientation of the mobile device (112) relative to the test element (114) by using sensor data of at least one sensor device (120) integrated into the mobile device (112);
b) subjecting the angular orientation of the mobile device (112) relative to the test element (114) to at least one validity test;
c) capturing at least one image (128) of at least a part of the test element (114) by using the camera (118), the at least one part of the test element (114) comprising at least one part of the test field (116); and
d) determining the concentration of the analyte in the bodily fluid from the image (128);
wherein at least one of steps c) and d) is performed by taking into account the result of the validity test in step b), wherein the method comprises retrieving an image stream by using the camera, wherein step c) comprises capturing one or more still images separately from the image stream by using a still image mode of the camera of the mobile device, wherein the at least one still image provides enhanced spatial resolution and/or higher dynamics compared to the images from the image stream, wherein at least a part of the images of the image stream are flagged with at least one item of information regarding whether the image was retrieved at a point in time for which the validity test determines the angular orientation of the mobile device relative to the test element to be valid, wherein the capturing of the still image is initiated automatically in case an image of the image stream was flagged with an item of information indicating a valid image at a point in time for which the image of the image stream was flagged as being valid, and wherein step d) is performed at least partially on the at least one still image.

2. The method according to the preceding claim, wherein the validity test in step b) comprises comparing the angular orientation of the mobile device (112) relative to the test element (114) with at least one target orientation, wherein the validity test determines the angular orientation of the mobile device (112) relative to the test element (114)
- to be valid in case the angular orientation deviates from the target orientation by no more than at least one predetermined angular tolerance; or
- to be invalid in case the angular orientation deviates from the target orientation by more than the at least one predetermined angular tolerance.

3. The method according to any one of the preceding claims, wherein the method carried out by the mobile device comprises monitoring the angular orientation of the mobile device (112) relative to the test element (114) and blocking the capturing of the at least one image (128) in case the validity test determines the angular orientation of the mobile device (112) relative to the test element (114) to be invalid, wherein the method further comprises unblocking the capturing of the at least one image (128) in case the validity test determines the angular orientation of the mobile device (112) relative to the test element (114) to be valid, wherein the method comprises automatically initiating the capturing of the at least one image (128) in case the validity test determines the angular orientation of the mobile device (112) relative to the test element (114) to be valid.

4. The method according to any one of the preceding claims, wherein step a) at least partially is performed under the assumption that the test element (114) is oriented in a predetermined orientation.

5. The method according to any one of the preceding claims, wherein the method further comprises determining an angular orientation of the test element (114) by placing the mobile device (112) on a support surface (130) and determining an angular orientation (132) of the support surface (130), wherein, in step a), the test element (114) is placed on the support surface (130).

6. The method according to any one of the preceding claims, wherein the method further comprises applying at least one sample of the bodily fluid to the test element (114), wherein step c) further comprises capturing at least one dry reference image of the at least one part of the test element (114) before applying the bodily fluid to the test element (114) and capturing at least one measurement image of the at least one part of the test element (114) after applying the bodily fluid to the test element (114), wherein, in step d), both the dry reference image and the measurement image are taken into account for determining the concentration of the analyte in the bodily fluid, wherein optionally steps a), b), and c) are performed at least once for capturing the dry image and at least once for capturing the measurement image.

7. The method according to any one of the preceding claims, wherein the method comprises providing user guidance for guiding a user towards a target orientation of the mobile device (112) relative to the test element (114).

8. The method according to any one of the preceding claims, wherein the method does not imply a mathematical correction of the at least one image (128) for angular misalignment.

9. A mobile device (112) having a camera (118), at least one sensor device (120) and at least one processor (126), the mobile device (112) being configured for determining a concentration of at least one analyte in a bodily fluid by using the test element (114), the mobile device (112) being configured to perform the method according to any one of claims 1, 2, 3, 5, 7 and 8.

10. A kit (118) comprising at least one mobile device (112) according to the preceding claim, the kit (110) further comprising at least one test element (114) having at least one test field (116).

11. The kit (110) according to the preceding claim, the kit (110) further comprising at least one reference card, the reference card having at least one reference color field.

12. A computer-readable storage medium comprising instructions which, when executed by the mobile device (112) of claim 9, cause the mobile device (112) to carry out the steps of the method according to any one of claims 1, 2, 3, 5, 7 and 8.

## Patentansprüche

1. Verfahren zum Bestimmen einer Konzentration von mindestens einem Analyten in einer Körperflüssigkeit durch Verwenden eines Mobilgeräts (112) und eines Testelements (114), wobei das Mobilgerät (112) eine Kamera (118) aufweist und das Testelement (114) mindestens ein Testfeld (116) aufweist, wobei das mit dem Mobilgerät ausgeführte Verfahren Folgendes umfasst
a) Bestimmen einer Winkelorientierung des Mobilgeräts (112) in Bezug auf das Testelement (114) durch Verwenden von Sensordaten von mindestens einer Sensorvorrichtung (120), die in dem Mobilgerät (112) integriert ist;
b) Unterziehen der Winkelorientierung des Mobilgeräts (112) in Bezug auf das Testelement (114) mindestens einem Gültigkeitstest;
c) Aufnehmen mindestens eines Bildes (128) von mindestens einem Teil des Testelements (114) durch Verwenden der Kamera (118), wobei der mindestens eine Teil des Testelements (114) mindestens einen Teil des Testfeldes (116) umfasst; und
d) Bestimmen der Konzentration des Analyten in der Körperflüssigkeit aus dem Bild (128);
wobei mindestens einer der Schritte c) und d) durchgeführt wird, indem das Ergebnis des Gültigkeitstests in Schritt b) berücksichtigt wird, wobei das Verfahren das Abrufen eines Bildstroms durch Verwenden der Kamera umfasst, wobei Schritt c) das Aufnehmen eines oder mehrerer Standbilder getrennt von dem Bildstrom durch Verwenden eines Standbildmodus der Kamera des Mobilgeräts umfasst, wobei das mindestens eine Standbild im Vergleich zu den Bildern aus dem Bildstrom eine bessere räumliche Auflösung und/oder höhere Dynamik liefert, wobei mindestens ein Teil der Bilder des Bildstroms mit mindestens einem Informationsdetail darüber, ob das Bild zu einem Zeitpunkt, für den der Gültigkeitstest die Winkelorientierung des Mobilgeräts in Bezug auf das Testelement für gültig erklärt, abgerufen wurde, markiert ist, wobei das Aufnehmen des Standbildes automatisch initiiert wird, wenn ein Bild des Bildstroms mit einem Informationsdetail markiert wurde, das zu einem Zeitpunkt, für den das Bild des Bildstromes als gültig markiert wurde, ein gültiges Bild angibt, und wobei Schritt d) mindestens teilweise an dem mindestens einen Standbild ausgeführt wird.

2. Verfahren nach dem vorstehenden Anspruch, wobei der Gültigkeitstest in Schritt b) das Vergleichen der Winkelorientierung des Mobilgeräts (112) in Bezug auf das Testelement (114) mit mindestens einer Zielorientierung umfasst, wobei der Gültigkeitstest die Winkelorientierung des Mobilgeräts (112) in Bezug auf das Testelement (114)
- für gültig erklärt, wenn die Winkelorientierung von der Zielorientierung um nicht mehr als mindestens eine vorbestimmte Winkeltoleranz abweicht; oder
- für ungültig erklärt, wenn die Winkelorientierung von der Zielorientierung um mehr als die mindestens eine vorbestimmte Winkeltoleranz abweicht.

3. Verfahren nach einem der vorstehenden Ansprüche, wobei das von dem Mobilgerät ausgeführte Verfahren das Überwachen der Winkelorientierung des Mobilgeräts (112) in Bezug auf das Testelement (114) und das Blockieren des Aufnehmens des mindestens einen Bildes (128) umfasst, wenn der Gültigkeitstest die Winkelorientierung des Mobilgeräts (112) in Bezug auf das Testelement (114) für ungültig erklärt, wobei das Verfahren ferner das Freigeben des Aufnehmens des mindestens einen Bildes (128) umfasst, wenn der Gültigkeitstest die Winkelorientierung des Mobilgeräts (112) in Bezug auf das Testelement (114) für gültig erklärt, wobei das Verfahren das automatische Initiieren des Aufnehmens des mindestens einen Bildes (128) umfasst, wenn der Gültigkeitstest die Winkelorientierung des Mobilgeräts (112) in Bezug auf das Testelement (114) für gültig erklärt.

4. Verfahren nach einem der vorstehenden Ansprüche, wobei Schritt a) mindestens teilweise unter der Annahme ausgeführt wird, dass das Testelement (114) in einer vorbestimmten Orientierung orientiert ist.

5. Verfahren nach einem der vorstehenden Ansprüche, wobei das Verfahren ferner das Bestimmen einer Winkelorientierung des Testelements (114) durch Platzieren des Mobilgeräts (112) auf einer Trägerfläche (130) und das Bestimmen einer Winkelorientierung (132) der Trägerfläche (130) umfasst, wobei in Schritt a) das Testelement (114) auf der Trägerfläche (130) platziert ist.

6. Verfahren nach einem der vorstehenden Ansprüche, wobei das Verfahren ferner das Aufbringen mindestens einer Probe von der Körperflüssigkeit auf das Testelement (114) umfasst, wobei Schritt c) ferner das Aufnehmen mindestens eines Trockenreferenzbildes von dem mindestens einen Teil des Testelements (114) vor dem Aufbringen der Körperflüssigkeit auf das Testelement (114) und das Aufnehmen mindestens eines Messbildes des mindestens einen Teils des Testelements (114) nach dem Aufbringen der Körperflüssigkeit auf das Testelement (114) umfasst, wobei in Schritt d) sowohl das Trockenreferenzbild als auch das Messbild für das Bestimmen der Konzentration des Analyten in der Körperflüssigkeit berücksichtigt werden, wobei gegebenenfalls die Schritte a), b) und c) mindestens einmal zum Aufnehmen des Trockenbildes und mindestens einmal zum Aufnehmen des Messbildes ausgeführt werden.

7. Verfahren nach einem der vorstehenden Ansprüche, wobei das Verfahren das Bereitstellen von Benutzerführung zum Führen eines Benutzers in Richtung einer Zielorientierung des Mobilgeräts (112) in Bezug auf das Testelement (114) umfasst.

8. Verfahren nach einem der vorstehenden Ansprüche, wobei das Verfahren keine mathematische Korrektur des mindestens einen Bildes (128) für Winkelversatz impliziert.

9. Mobilgerät (112) mit einer Kamera (118), mindestens einer Sensorvorrichtung (120) und mindestens einem Prozessor (126), wobei das Mobilgerät (112) für das Bestimmen einer Konzentration von mindestens einem Analyten in einer Körperflüssigkeit durch Verwenden des Testelements (114) ausgebildet ist, wobei das Mobilgerät (112) dafür ausgebildet ist, das Verfahren nach einem der Ansprüche 1, 2, 3, 5, 7 und 8 auszuführen.

10. Kit (118), umfassend mindestens ein Mobilgerät (112) nach dem vorstehenden Anspruch, wobei der Kit (110) ferner mindestens ein Testelement (114) mit mindestens einem Testfeld (116) umfasst.

11. Kit (110) nach dem vorstehenden Anspruch, wobei der Kit (110) ferner mindestens eine Referenzkarte umfasst, wobei die Referenzkarte mindestens ein Referenzfarbfeld aufweist.

12. Computerlesbares Speichermedium, das Anweisungen umfasst, die bei der Ausführung von dem Mobilgerät (112) nach Anspruch 9 veranlassen, dass das Mobilgerät (112) die Schritte des Verfahrens nach einem der Ansprüche 1, 2, 3, 5, 7 und 8 ausführt.

## Revendications

1. Procédé de détermination d'une concentration d'au moins un analyte dans un fluide corporel en utilisant un appareil mobile (112) et un élément de test (114), l'appareil mobile (112) ayant une caméra (118), et l'élément de test (114) ayant au moins un champ de test (116), le procédé réalisé par l'appareil mobile comprenant
a) la détermination d'une orientation angulaire de l'appareil mobile (112) par rapport à l'élément de test (114) en utilisant des données de capteur d'au moins un appareil capteur (120) intégré dans l'appareil mobile (112) ;
b) la soumission de l'orientation angulaire de l'appareil mobile (112) par rapport à l'élément de test (114) à au moins un test de validité ;
c) la capture d'au moins une image (128) d'au moins une partie de l'élément de test (114) en utilisant la caméra (118), l'au moins une partie de l'élément de test (114) comprenant au moins une partie du champ de test (116) ; et
d) la détermination de la concentration de l'analyte dans le fluide corporel à partir de l'image (128) ;
dans lequel au moins l'une des étapes c) et d) est exécutée en prenant en compte le résultat du test de validité de l'étape b), dans lequel le procédé comprend la récupération d'un flux d'images en utilisant la caméra, dans lequel l'étape c) comprend la capture d'une ou de plusieurs images fixes séparément du flux d'images en utilisant un mode d'image fixe de la caméra de l'appareil mobile, dans lequel l'au moins une image fixe fournit une résolution spatiale améliorée et/ou une meilleure dynamique par comparaison aux images du flux d'images, dans lequel au moins une partie des images du flux d'images sont marquées avec au moins un élément d'information pour savoir si l'image a été récupérée à un point de temps pour lequel le test de validité détermine que l'orientation angulaire de l'appareil mobile par rapport à l'élément de test est valide, dans lequel la capture de l'image fixe est initiée automatiquement dans le cas où une image du flux d'images a été marquée avec un élément d'information indiquant une image valide à un point de temps pour lequel l'image du flux d'images a été marquée comme étant valide, et dans lequel l'étape d) est exécutée au moins partiellement sur l'au moins une image fixe.

2. Procédé selon la revendication précédente, dans lequel le test de validité de l'étape b) comprend la comparaison de l'orientation angulaire de l'appareil mobile (112) par rapport à l'élément de test (114) avec au moins une orientation cible, dans lequel le test de validité détermine que l'orientation angulaire de l'appareil mobile (112) par rapport à l'élément de test (114)
- est valide dans le cas où l'orientation angulaire dévie de l'orientation cible de pas plus d'au moins une tolérance angulaire prédéterminée ; ou
- est invalide dans le cas où l'orientation angulaire dévie de l'orientation cible de plus de l'au moins une tolérance angulaire prédéterminée.

3. Procédé selon l'une quelconque des revendications précédentes, dans lequel le procédé réalisé par l'appareil mobile comprend la surveillance de l'orientation angulaire de l'appareil mobile (112) par rapport à l'élément de test (114) et le blocage de la capture de l'au moins une image (128) dans le cas où le test de validité détermine que l'orientation angulaire de l'appareil mobile (112) par rapport à l'élément de test (114) est invalide, dans lequel le procédé comprend en outre le déblocage de la capture de l'au moins une image (128) dans le cas où le test de validité détermine que l'orientation angulaire de l'appareil mobile (112) par rapport à l'élément de test (114) est valide, dans lequel le procédé comprend l'initiation automatique de la capture de l'au moins une image (128) dans le cas où le test de validité détermine que l'orientation angulaire de l'appareil mobile (112) par rapport à l'élément de test (114) est valide.

4. Procédé selon l'une quelconque des revendications précédentes, dans lequel l'étape a) est au moins partiellement exécutée en supposant que l'élément de test (114) est orienté dans une orientation prédéterminée.

5. Procédé selon l'une quelconque des revendications précédentes, dans lequel le procédé comprend en outre la détermination d'une orientation angulaire de l'élément de test (114) en plaçant l'appareil mobile (112) sur une surface de support (130) et en déterminant une orientation angulaire (132) de la surface de support (130), dans lequel, à l'étape a), l'élément de test (114) est placé sur la surface de support (130).

6. Procédé selon l'une quelconque des revendications précédentes, dans lequel le procédé comprend en outre l'application d'au moins un échantillon du fluide corporel sur l'élément de test (114), dans lequel l'étape c) comprend en outre la capture d'au moins une image de référence sèche de l'au moins une partie de l'élément de test (114) avant l'application du fluide corporel sur l'élément de test (114) et la capture d'au moins une image de mesure de l'au moins une partie de l'élément de test (114) après l'application du fluide corporel sur l'élément de test (114), dans lequel, à l'étape d), l'image de référence sèche et l'image de mesure sont toutes deux prises en compte pour déterminer la concentration de l'analyte dans le fluide corporel, dans lequel éventuellement les étapes a), b) et c) sont exécutées au moins une fois pour la capture de l'image sèche et au moins une fois pour la capture de l'image de mesure.

7. Procédé selon l'une quelconque des revendications précédentes, dans lequel le procédé comprend la fourniture de conseils à l'utilisateur pour guider un utilisateur vers une orientation cible de l'appareil mobile (112) par rapport à l'élément de test (114).

8. Procédé selon l'une quelconque des revendications précédentes, dans lequel le procédé n'implique pas une correction mathématique de l'au moins une image (128) pour un mauvais alignement angulaire.

9. Appareil mobile (112) ayant une caméra (118), au moins un appareil capteur (120) et au moins un processeur (126), l'appareil mobile (112) étant conçu pour déterminer une concentration d'au moins un analyte dans un fluide corporel en utilisant l'élément de test (114), l'appareil mobile (112) étant conçu pour exécuter le procédé selon l'une quelconque des revendications 1, 2, 3, 5, 7 et 8.

10. Kit (118) comprenant au moins un appareil mobile (112) selon la revendication précédente, le kit (110) comprenant en outre au moins un élément de test (114) ayant au moins un champ de test (116).

11. Kit (110) selon la revendication précédente, le kit (110) comprenant en outre au moins une carte de référence, la carte de référence ayant au moins un champ coloré de référence.

12. Support de stockage lisible par ordinateur comprenant des instructions qui, lorsqu'elles sont exécutées par l'appareil mobile (112) selon la revendication 9, poussent l'appareil mobile (112) à réaliser les étapes du procédé selon l'une quelconque des revendications 1, 2, 3, 5, 7 et 8.
